(19) Europäisches Patentamt / European Patent Office / Office européen des brevets

(11) **EP 4 085 820 B1**

(12) **EUROPEAN PATENT SPECIFICATION**

(45) Date of publication and mention
of the grant of the patent:
**17.07.2024 Bulletin 2024/29**

(21) Application number: **21172136.0**

(22) Date of filing: **04.05.2021**

(51) International Patent Classification (IPC):
*A61B 5/00* (2006.01)      *A61B 5/145* (2006.01)

(52) Cooperative Patent Classification (CPC):
**A61B 5/0095; A61B 5/1451; A61B 5/14532;**
**A61B 5/4839; A61B 5/7246**

(54) **PROCESS FOR ANALYZING A TISSUE**

VERFAHREN ZUR GEWEBEANALYSE

PROCÉDÉ D'ANALYSE D'UN TISSU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.11.2022 Bulletin 2022/45**

(73) Proprietor: **Eclypia
38000 GRENOBLE (FR)**

(72) Inventors:
• **BLANC, Romain
38000 GRENOBLE (FR)**
• **COUTARD, Jean-Guillaume
38660 SAINT PANCRASSE (FR)**
• **GALLEGOS, Alexandre
75002 Paris (FR)**
• **LE ROUX-MALLOUF, Thibault
38000 GRENOBLE (FR)**
• **RUNGET, Freddy
38100 GRENOBLE (FR)**

(74) Representative: **Fidal Innovation
4-6 avenue d'Alsace
92400 Courbevoie (FR)**

(56) References cited:
WO-A1-2020/094233     US-A1- 2009 292 202
US-A1- 2018 323 571     US-A1- 2021 052 164
US-A1- 2021 109 019

**Description**

**FIELD OF THE INVENTION**

The present invention relates to a process for non-invasively analyzing a target.

More specifically, the invention relates to a process for analyzing a human tissue or an animal tissue, and further for measuring a substance in this tissue, for example for measuring blood glucose.

**TECHNOLOGICAL BACKGROUND**

425 million people have diabetes, including type 1 and type 2, worldwide. If the blood glucose (blood sugar) level of diabetic patients is not maintained, they may have serious complications, such as cardiovascular disease, kidney disease, and diabetic foot. Therefore, the blood glucose level should be regularly monitored and high blood glucose levels need to be addressed promptly.

The blood glucose levels may be determined from blood samples obtained invasively. Obtaining the blood by pricking a finger can be very inconvenient for a diabetic patient who needs to have their blood sugar measured several times a day, and there is further a risk of infection.

Therefore, non-invasive blood glucose measurement methods are preferred when possible.

Known methods for the measurement of blood sugar in a non-invasive manner are described in the following publication : Photoacoustic techniques, as described in Allan Rosencwaig and Allen Gersho : "Theory of the photoacoustic effect with solids", Journal of Applied Physics 47, 64 (1976), rely upon the irradiation of a target with light, such as light provided by a laser beam.

The irradiation produces thermal effects in the target, such as a volumetric expansion and the thin layer of air contacting the target due to thermal diffusion, which causes a pressure oscillation that generates an acoustic wave.

The characteristics of this acoustic wave depend upon several factors, such as the target's absorption coefficient to the wavelength of light used, the density of the medium through which the acoustic wave propagates, the thermal expansion coefficient of the target, the velocity of the acoustic wave, and so on.

If skin is used as a target, the light may penetrate a distance into the skin and excite molecules, such as glucose molecules, beneath the skin. The acoustic wave generated by the thermal excitation (and subsequent volumetric expansion) of these glucose molecules can be used to estimate the concentration of the glucose molecules.

Kottmann J, Rey JM, Sigrist MW. "Mid-Infrared Photoacoustic Detection of Glucose in Human Skin : Towards Non-Invasive Diagnostics". Sensors (Basel). 2016;16(10):1663. Published 2016 Oct 10 describes a non invasive glucose-monitoring system based on the Mid Infra-Red (MIR) photoacoustic (PA) detection.

This document focuses on the improvement of the measurement sensitivity and stability.

Indeed, measurements based on photoacoustic detection suffer from several influences that can't be controlled in-vivo : during in vivo studies, not only the transdermal water loss through passive diffusion but also transpiration provokes the evaporation of water into the coupling gas of the PA chamber. The measurement stability and the sensitivity are improved by using two different laser beams that are modulated at a particular modulation frequency.

However, using two lasers for every measurement results in high power consumption, which isn't compatible with the portable nature of the device.

Further, measurements are influenced not only by the temperature and the relative humidity but also by the structure of the skin reached by the laser beam.

Kottmann points out the known theoretical relationship (established by Rosencwaig) between the modulation frequency and the depth of material reached by the laser beam, in case of a homogeneous material. Unfortunately, the skin cannot be considered as a homogeneous material. Even more annoying, skin is a living material, which changes over time.

The skin structure depends on the patient, and for a given patient, it depends among others on the pressure exerted on the skin, on the temperature and on the water content of skin. It can also evolve due to disquamation for example,etc.

For an analyte sensor to be reliable, it is of particular importance to ensure that the measurement is always taken in the same layer of the skin, and that this layer contains the analyte monitor, even if the depth of this layer evolves from time to time. In this case and only in this case can a measurement be analyte-specific, and a reproducible analyte value can be deduced from the measurement.

For example, US2021/0052164 describes a sensor for non-invasive optoacoustic measurements of skin, comprising acquiring quantitative information at different depths which is not sufficient to ensure that a particular layer containing the analyte of interest is monitored. US2021/0109019 discloses the use of several modulation frequencies to obtain specific information at different depths under the surface of a target. However, once again, nothing allows to associate a depth with a particular layer of the target containing the analyte of interest.

US 10,261,011 B2 describes a glucose-monitoring device comprising an excitation laser beam and a measuring laser beam. A time-dependent response signal from the measuring beam is detected. The intensity distribution of the time-dependent response signal is a function of the depth under the skin surface and is studied at several modulation frequencies of the excitation beam. The excitation beam is modulated with different frequencies between 10 Hz and 500 Hz.

WO2020/094265A1 also describes a glucose-monitoring device comprising an excitation laser beam and a measuring laser beam. Several response signal curves can be determined one after the other using different modulation frequencies of the excitation transmission device. From the plurality of response signal curves at different modulation frequencies, an information specific to a depth zone may be obtained.

However, neither WO2020/094265A1 nor US 10,261,011 B2, solves the problem of the in-vivo measurement repeatability and analyte-specificity despite skin structure variations, which means ensuring that the same skin zone (and not the same depth) is monitored, and that this zone indeed contains the analyte to be monitored.

One can note that measurement repeatability isn't either addressed by invasive analyte-monitoring systems, since the needle depth can change between two samplings and even if it didn't, skin structure can change over time.

Therefore, the process according to the invention aims at providing an analysis of the structure of a target over time, in particular providing information on both the invariants and the changes in the structure of a target over time.

In a second aspect, the process aims at improving the repeatability and the analyte-specificity of in-vivo analyte measurement at sustainable energy and time costs, for a portable non-invasive analyte sensor based on photoacoustic detection.

The analyte is possibly blood glucose but other analytes may be addressed.

## SUMMARY OF THE INVENTION

The invention relates to a process for non-invasively analyzing a target with an analysis apparatus comprising:

- an intensity-modulation device, a light emitter emitting an intensity-modulated light, the modulation frequency being controllable,
- at least one detection cell comprising a sensor sensing directly or indirectly a thermal wave propagating out of the target in response to an irradiation of said target by the light emitted,
- a processor module configured to receive and process sensor data from at least one detection cell.

The process comprises :

a. carrying out at least one irradiation of the target with the light emitter in a first mode, the modulation frequency of the intensity of the light emitted being swept during each of the at least one irradiation in the first mode over a frequency sweep range, and the processor module receiving sensor data from a detection cell for each of the at least one irradiation in the first mode,

b. carrying out at least one additional irradiation of said target with said light emitter in said first mode, the processor module receiving sensor data from a detection cell for each of the at least one additional irradiation in the first mode,

c. processing with the processor module the sensor data received for at least two irradiations in the first mode and forming with the processor module a time series comprising sensor data for each of a plurality of discrete frequencies in the frequency sweep range,

d. calculating, using the processor module, a discrete mode modulation frequency based on an analysis of the time series comprising sensor data for each of a plurality of discrete frequencies in the frequency sweep range, said analysis comprising forming a time series for each of a plurality of discrete frequencies and calculating at least one value indicative of the similarity or the dissimilarity between two time series at two of the plurality of discrete frequencies,

e. carrying out at least one irradiation of the target with a light emitter emitting an intensity-modulated light in a second mode, the modulation frequency of the intensity of the light emitted in the second mode being the discrete mode modulation frequency calculated at step d, the processor module receiving sensor data from a detection cell for each of the at least one irradiation in the second mode,

f.calculating, using the processor module, an analyte value based on the sensor data received for at least one of the irradiation in the second mode.

Thanks to this arrangement, for each irradiation in the first mode, the target is irradiated with a light which intensity is modulated, the modulation frequency being swept in a sweep range.

As the modulation frequency decreases, the light penetrates deeper into the target and the thermal wave sensed, directly (for example with a thermal sensor) or indirectly (for example with an electroacoustic sensor sensing the acoustic wave corresponding to the thermal wave) in response to this light provides information on deeper parts of the target.

Each irradiation in the first mode provides as a consequence information on the structure along the depth of the target. Since at least two successive irradiations at two different times are performed, a time series can be formed. The processing of the time series provides information on the changes and invariants in the target structure along the depth.

The time series formed with the data acquired over the whole sweep range can for example be divided into a plurality of time series, each of them concerning a discrete frequency or an interval of frequencies in the sweep range and as a consequence, each of them concerning a zone of the target at a particular distance or interval of distances form the light emitter. It is then possible to compare two or more of the plurality of time series in order to detect different stable or changing zones or interfaces in the target.

.

In this way, an optimal modulation frequency for a further irradiation, intended to reach a particular zone of a target, can be set, taking into account the invariants and/or the changes in the target structure.

Thanks to this arrangement, if an analyte is to be measured always in a particular layer, this layer having a changing depth in the target, the optimal modulation frequency ensuring that this layer is reached is adapted according to the results of the first mode irradiations and the target is irradiated with a light having an intensity modulated at this optimal modulation frequency. The sensor data acquired in this second mode are in consequence analyte-specific, and the measure of the analyte is analyte-specific and repeatable.

In one embodiment, in the process for non-invasively analyzing a target with an analysis apparatus, a first sensor is provided for carrying out steps a and b and a second sensor is provided for carrying out step e.

In this manner, one sensor can be dedicated to the first mode acquisitions and another one to the second mode acquisition. It is even possible to use different type of sensors for the first mode acquisitions and the second mode acquisitions, depending for example on the accuracy required for each mode.

In one embodiment, in the process for non-invasively analyzing a target with an analysis apparatus, a second light emitter emitting an intensity-modulated light at a controllable modulation frequency is provided for carrying the at least one irradiation at step e.

In this manner, one light emitter can be dedicated to the first mode acquisitions and another one to the second mode acquisitions. It is even possible to use different type of light emitters for the first mode acquisitions and the second mode acquisitions, depending for example on the power or the quality of beam required for each mode.

In one embodiment, at step e of the process for non-invasively analyzing a target with an analysis apparatus, at least two irradiations in the second mode are performed, two successive irradiations in the second mode being separated by a first period of time TDM.

This arrangement enables to carry out several successive irradiations in the second mode and in consequence to measure repeatedly an analyte with the same modulation frequency. The first mode irradiations are indeed time- and power-consuming, and if it is found that the characteristic time of changes in the target is longer than the period of time between two measurements of the analyte, it is more efficient not to carry out a first mode irradiation after or before each second mode irradiation.

In one embodiment, at step f of the process for non-invasively analyzing a target with an analysis apparatus, the analyte value is calculated based on the sensor data received for a plurality of the at least two irradiations in the second mode at the preceding step e.

This makes it possible to smooth the analyte value, for example by calculating an average value, a moving average value, etc., of analyte values measured at close time points. The accuracy or the confidence interval of the measurement can thus be improved.

In one embodiment, steps b, c, d, and e of the process for non-invasively analyzing a target with an analysis apparatus are periodically repeated in this order, two successive steps b being separated by a second period of time TSM.

In this manner, if the characteristic time of changes in the target is called T, TSM can be chosen so as to be smaller than T so that the optimal modulation frequency of the intensity of the light emitter for the analysis to be performed is recalculated often enough to ensure that this the optimal modulation frequency is consistent with the current structure of the target.

In one embodiment, in the process for non-invasively analyzing a target with an analysis apparatus, the first period of time TDM is shorter than said second period of time TSM.

This permits both to carry out several second mode acquisitions between two first mode acquisitions (this being more efficient from a time and power point of view), and to adapt the modulation frequency for the second mode often enough to ensure the specificity and the repeatability of the measurements in the second mode.

In one embodiment of the process for non-invasively analyzing a target with an analysis apparatus, a first integer N is predetermined and at step c, the time series is formed with the data acquired during the N latest irradiations in the first mode.

This allows to analyze the latest changes or invariants in the target structure. If N' first mode irradiations have been performed, N' being much higher than N ( N'>> N), the impact of the last measurement in the time series comprising N' points will be negligible whereas it can be significant in the time series comprising N points.

In one embodiment, at step a of the process for non-invasively analyzing a target with an analysis apparatus, N-1 irradiations are performed in the first mode, two successive irradiations of step a being separated by a third period of time TDM.

This makes it possible among others to carry out an initialization phase over a controlled duration : the initialization phase will last approximately (N-1)*TDM and it will thus be short enough if TDM is short enough.

In this case, the time series formed with the data acquired over the whole sweep range is divided into a plurality of time series, each of them concerning a discrete frequency or an interval of frequencies in the sweep range and as a consequence, each of them concerning a zone of the target at a particular distance or interval of distances form the light emitter. It is then possible to compare two or more of the plurality of time series in order to detect different steady or changing zones in the target.

For example, if two time series corresponding to two successive frequencies are found similar, one can infer that the light modulated at both of these modulation frequencies reaches a same zone of the target.

In one embodiment, at step d of the process for non-invasively analyzing a target with an analysis apparatus , the analysis of the time series comprising sensor data for each of a plurality of discrete frequencies in the frequency sweep range further comprises performing a dynamic time warping algorithm and/or a correlation analysis on this time series.

Such analyzes are fast and effective means to gain insight into the target structure, and in particular on its changes and invariants over time, each type of analysis giving specific insight.

In one embodiment, in the process for non-invasively analyzing a target with an analysis apparatus, an intensity-modulated light comprises at least one wavelength that is absorbed by an analyte to be measured in the target.

Thanks to this arrangement, the sensor data is informative of the analyte concentration in the target.

In one embodiment of the process for non-invasively analyzing a target with an analysis apparatus, the at least one wavelength that is absorbed by an analyte to be measured in the target is within the mid infrared range.

In case of a human or animal tissue, many analytes of interest absorb in the mid infrared range, for example glucose. This arrangement thus allows to non-invasively monitor blood glucose with improved repeatability.

In one embodiment, in the process for non-invasively analyzing a target with an analysis apparatus, the analysis apparatus further comprises a user interface or is further configured to transmit data to a medicament delivery device. At step d, the results of the analysis of the time series comprising sensor data for each of a plurality of discrete frequencies in the frequency sweep range are compared to a predetermined threshold, and, based on the results of this comparison, an alarm signal is displayed on the user interface or transmitted to the medicament delivery device.

In this case, an unexpected change in the target structure is interpreted as a failure either of the analysis apparatus or of its interaction with the target. The user may, thanks to the signal displayed on the interface, check the analysis apparatus. If a therapy is delivered based on the data provided by the analysis apparatus, a safety process may be triggered for this delivery.

In one embodiment, in the process for non-invasively analyzing a target with an analysis apparatus, at least one detection cell comprises a sensor is chosen in the list { electroacoustic sensor, thermal sensor}.

A thermal sensor directly senses the thermal wave caused by the irradiation. An electroacoustic sensor indirectly senses the thermal wave caused by the irradiation, in that it detects the acoustic wave that forms in a gas around the target due to the thermal wave reaching the surface of the target.

The invention also relates to an apparatus for non-invasively analyzing a target comprising:

- at least one intensity-modulation device, a light emitter emitting an intensity-modulated light at a controllable modulation frequency,
- at least one detection cell comprising a sensor sensing directly or indirectly a thermal wave propagating out of the target in response to an irradiation of said target by the light emitted,
- at least one processor module configured to receive and process sensor data from at least one detection cell.

In this apparatus, at least one light emitter is configured to :

- carry out at least one irradiation of the target with the light emitter in a first mode, the modulation frequency of the intensity of the light emitted being swept during each of the at least one irradiation in the first mode over a frequency sweep range,
- carry out at least one additional irradiation of said target with said light emitter in the first mode,

and at least one processor module is configured to :

- receive sensor data from at least one detection cell for each of the at least one irradiation in a first mode,
- process the sensor data received for at least of the at least two irradiations in the first mode and form a time series comprising sensor data for each of a plurality of discrete frequencies in the frequency sweep range, and

- calculate a discrete mode modulation frequency based on an analysis of the time series comprising data for each of a plurality of discrete frequencies in the frequency sweep range, said analysis comprising forming a time series for each of a plurality of discrete frequencies and calculating at least one value indicative of the similarity or the dissimilarity between two time series at two of the plurality of discrete frequencies,
- at least one light emitter is configured to carry out at least one irradiation of the target with the light emitter in a second mode, the light emitted in the second mode being intensity-modulated at said discrete mode modulation frequency calculated by at least one processor module,
- at least one processor module is configured to receive sensor data from at least one detection cell for each of the at least one irradiation in the second mode and to calculate an analyte value based on the sensor data received for at least one irradiation in the second mode.

In one embodiment, the apparatus for non-invasively analyzing a target is wearable.

In this case, it is possible to continuously analyze a human or animal tissue. For example, this apparatus can be used as a continuous glucose monitor.

According to another aspect, the invention relates to a system comprising the apparatus for non-invasively analyzing a target and a medicament delivery device, the apparatus for non-invasively analysing a target being further configured to send data to the medicament delivery device and the system is further configured to determine a medicament therapy to be delivered by the medicament delivery device based on at least one analyte value calculated with the processor module of the apparatus for non-invasively analyzing a target.

This allows to constitute either an open-loop system or a closed-loop system for delivering a medicament therapy, for example an insulin therapy.

**BRIEF DESCRIPTION OF THE DRAWINGS**

Embodiments of the invention will be described below, in relation to the following drawings :

Fig. 1 shows a schematic of an analyte monitor in accordance with exemplary embodiments.

Fig. 2 is a schematic view of the skin structure.

Fig. 3 is a chronogram showing the switching between the two measurement modes in an exemplary embodiment of the process.

Fig. 4a is a theoretical example of obtaining a similarity-dissimilarity-matrix "over time", illustrating the differences with an "instantaneous" matrix.

Fig. 4b shows the results of an experiment carried out on an anesthetized pig, with 106 first mode irradiations, each irradiation lasting one minute, the whole experiment lasting around two hours. Fig 4b, 1 shows the similarity-dissimilarity matrix "over time" for the first 53 time points, and fig 4b,2 shows the similarity-dissimilarity matrix "over time" for the last 53 time points.

Fig. 5 is an example of similarity-dissimilarity-matrix "over time" obtained in a first experiment with a first comparison technique.

Fig. 6 shows an example of similarity-dissimilarity-matrix "overtime" obtained in a second experiment with 60 first mode irradiations, each irradiation lasting one minute, with a sweep range [100 Hz, 13 800 kHz] and in which no zone can be identified.

Fig. 7a and 7b show the results of an experiment carried out on an anesthetized pig, with 130 first mode irradiations, each irradiation lasting one minute, with a sweep range [100 Hz, 2300 kHz]. Fig 7a shows a correlation matrix, the correlation coefficient (ranging from -1 to 1) being represented both by its value and a color. Fig 7b shows a DTW matrix, the distance (ranging from 0 to 30 in this case) being represented with a color scale.

Fig. 8a and 8b show the results of an experiment carried out on an anesthetized pig, with 60 first mode irradiations, each irradiation lasting one minute, with a sweep range [100 HZ, 13 800 kHz]

On the drawings, the same reference signs show the same or similar objects.

## DETAILED DESCRIPTION

The invention deals with a non-invasive analysis apparatus and a process for analyzing a target with the analysis apparatus. The analysis apparatus 1 may be used to analyze a target 2 and/or to measure a substance or analyte in a target 2, for example a human or an animal tissue, like skin.

For example, the substance or analyte to be measured is blood glucose. More specifically, a blood glucose value can be estimated based on a non-invasive interstitial glucose measurement.

The analysis apparatus 1 can be portable or wearable.

The analysis apparatus 1 comprises at least :

- an intensity-modulation device, a light emitter emitting an intensity-modulated light, a light emitter controller controlling at least a modulation frequency at which said intensity-modulation device modulates the intensity of a light emitted by the light emitter,
- at least one detection cell 12 comprising a sensor sensing directly or indirectly a thermal wave propagating out of the target in response to an irradiation of said target by the light emitted,
- a processor module 13 configured to receive and process sensor data from at least one detection cell.

In an exemplary embodiment, the detection cell 12 is a photoacoustic (PA) detection cell.

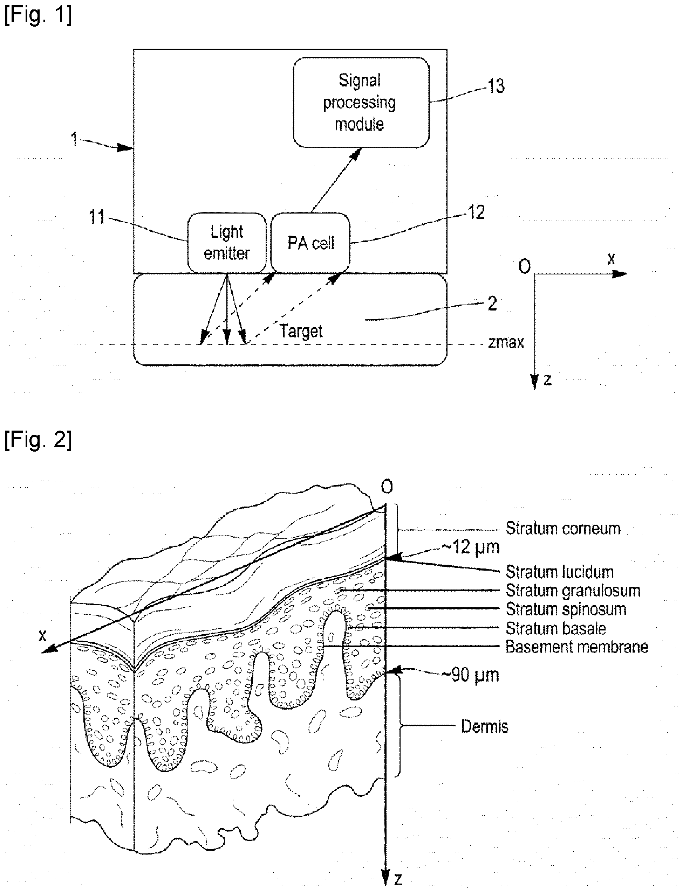

Fig. 1 shows a schematic diagram illustrating how photoacoustic measurement techniques may be used to obtain non-invasively an analyte sensor signal representative of an analyte concentration, for example a blood glucose concentration level of a user.

As can be seen on Fig. 1, a light emitter block 11 may comprise a light emitter configured to emit a light beam towards a target 2. The target could be a patient's tissue, for example skin.

The light incident on the target 2 penetrates a distance into the target 2 and interacts with analyte molecules that are present inside the target 2, for example glucose molecules.

The analyte molecules are in consequence excited thermally.

As a result, a thermal wave propagates in the target 2, in particular toward the surface of the target 2 and, then into the medium surrounding the target 2, for example air.

In case a gaseous medium is surrounding the target 2, an acoustic wave associated with the thermal wave propagates in this gaseous medium surrounding the target 2

Consequently, the thermal wave propagating in response to the irradiation of the target 2 can be directly or indirectly sensed.

In the first case, the thermal wave can be sensed with a thermal sensor comprised in the detection cell 12.

In the second case, one can for example use a photoacoustic sensor to sense the acoustic wave associated with the thermal wave in a gaseous medium surrounding the target 2.

The propagation of light and of the acoustic wave is schematically illustrated in Fig. 1 through the use of respectively bold and dashed arrows.

In case the acoustic wave is sensed by a photoacoustic detection cell 12, this detection cell comprises a chamber filled with a gas (for example air) through which the acoustic wave propagates and one or more appropriate sensors placed in this chamber, for example opposite the target 2.

In an exemplary embodiment, the photoacoustic detection cell 12 comprises an electroacoustic sensor configured to convert the pressure of the acoustic wave into an electrical signal, such as a microphone. In an alternative exemplary embodiment, the photoacoustic detection cell 12 comprises a transducer, for example a piezoelectric transducer.

In exemplary embodiments, the electroacoustic sensor is operably connected to a signal processing module 13. The signal processing module 13 comprises an analog-to-digital converter configured to convert the analog electrical signal from the electroacoustic sensor to a digital signal.

The signal processing module 13 optionally comprises an operational amplifier operably connected to the analog-to-digital converter and configured to further amplify the electronic signal derived from the acoustic response of the target 2.

In exemplary embodiments, the analog-to-digital converter is operably connected to a digital signal processor for processing of the digital signal.

In a particular embodiment, the light emitter emits an intensity-modulated laser beam at at least one particular wavelength towards the target 2.

In an exemplary embodiment, the light emitter is a light-emitting diode (LED).

In an alternative embodiment, the light emitter is a laser chip.

For example, the light emitter comprises a Quantum Cascade Laser (QCL) emitting in the mid-infrared region (MIR-QCL).

The light emitter block 11 also comprises the circuitry associated with the light emitter and a controller module configured to control the light emitter such that the intensity of the light emitted by the light emitter is modulated at a tunable intensity modulation frequency. The intensity modulation frequency is called $f_{mod}$ in the rest of the description.

The intensity modulation can be obtained by any known electric or mechanic means.

The light can be emitted continuously or in a pulsed manner.

In a particular embodiment, the light emitter controller module is configured to constrain the light emitter so as to emit light pulses. The pulses may have durations of about 100 ns to 500 ns per pulse, and a duty cycle for example of 1% to 20%, which corresponds to a frequency of the order of 20 kHz to 2 MHz.

It must be understood that, in order to help understand the invention, a blood glucose sensor will often be described in the detailed examples below, but another analyte can be measured in the same way by merely adapting the wavenumber of the laser beam to the analyte to be monitored.

The process and system described here can also be adapted to other targets or tissues than skin. For example, liquid targets such as saliva could be analyzed.

In an exemplary embodiment, at least one wavelength λirrad of the light emitted by the light emitter is selected so as the light strongly interacts with an analyte of interest.

In case an analyte of interest is glucose, the wavelength of the light emitted by the light emitter can be selected to correspond to wavelengths that interact strongly with glucose molecules for thermal excitation of the glucose molecules.

For example, light having wavenumbers (the reciprocal of wavelength) of between about 1150 cm$^{-1}$ and 1000 cm$^{-1}$ interact strongly with glucose molecules. This wavenumber range corresponds to wavelengths in the mid-infrared (MIR) region.

Preferably, but not mandatorily, the analysis apparatus directly contacts the target 2. For example, part of the detection chamber of a photoacoustic detection cell 12 is in direct contact with skin.

In common ranges of wavelength λirrad (such as MIR), the light beam travels in straight line through the target 2, without light scattering but being progressively absorbed by the target.

The photons of the irradiation light beam are absorbed by the molecules that can absorb light at the chosen wavelength (Zirrad).

In vivo, the absorbing chemical molecules in the mid-infrared region comprise for example water, hemoglobin, melanin, lipids, other proteins such as collagen and elastin, and of course glucose.

Due to the absorption phenomenon, the intensity of the irradiation light beam decreases as it goes deeper in the target, following approximately an exponential law. The optical penetration depth ($\mu_a$) can be defined as a characteristic length of this exponential law.

The absorption of light by a given material is characterized by this characteristic absorption length, or optical penetration depth ($\mu_a$). According to a convention, 67% of the light energy is absorbed by the portion of target 2 located at less than the optical penetration depth of the entry surface of the target.

For a given material, $\mu_a$ is mostly a function of the wavelength λirrad. The light absorption coefficient $\alpha(\lambda irrad)$, which is the inverse of $\mu_a(\lambda irrad)$, is also mostly a function of the wavelength λirrad.

In case of skin, the light absorption coefficient $\alpha(\lambda irrad)$ is as a first approximation proportional to the concentration of molecules absorbing at λirrad, for example glucose at wavenumbers of between about 1150 cm$^{-1}$ and 1000 cm$^{-1}$.

As a consequence of the absorption phenomenon, the target undergoes thermal excitation, associated with a thermal wave having the same frequency as the intensity modulation frequency $f_{mod}$ of the light beam.

If the thermal wave propagates through the target 2 towards the air surrounding the target, it can either be directly detected with a thermal sensor close to the external surface of the target 2, or it can result in a pressure wave at the modulation frequency, that can be detected in the air surrounding the target 2, for example with an electroacoustic sensor comprised in a photoacoustic cell 12.

The thermal diffusion of the thermal wave is characterized by a characteristic diffusion length, called thermal diffusion length ($\mu_s$).

As a first approximation, the relationship between the thermal diffusion length ($\mu_s$) and the modulation frequency of the intensity-modulated light beam is expressed by the following formula (1):

[Formula 1]

$$\mu_s = \sqrt{\frac{D}{\pi \, f_{mod}}}$$

where D is the thermal diffusivity of the target.

In the case of photoacoustic detection in solid targets, such as human or animal tissues, as described by the Rosencwaig-Gersho model, the amplitude of the photoacoustic signal $S_{PA}$ is proportional to the absorption coefficient $\alpha(\lambda irrad)$ (and hence to the glucose content of skin in case of blood glucose detection) only where the thickness L of the target, $\mu_s$ and $\mu_a$ fulfill the requirement of the following formula (2) :

[Formula 2]

$$\mu_s < \mu_a \ll L$$

In a theoretical case, considering an homogeneous solid, the amplitude of the photoacoustic signal $S_{PA}$ follows the following formula (3) :

[Formula 3]

$$S_{PA} \propto \frac{\alpha(\lambda irrad).\,I_0(\lambda irrad).\,P_0}{V.\,f_{mod}^{\frac{3}{2}}}$$

where $I_0(\lambda irrad)$ represents the incident light intensity, V the enclosed gas volume of the photoacoustic detection cell with gas pressure $P_0$.

The requirement of a large sample thickness L is automatically filled since the absorption depth in skin is small. The additional requirement ($\mu_s < \mu_a$) can be fulfilled by appropriately choosing the modulation frequency $f_{mod}$ of the laser beam.

In this case, the signal detected by the detection cell 12 is limited by the thermal diffusion length. In other words, the photoacoustic signal detected is indicative of the target portion below its external surface and up to the thermal diffusion length, which depends mainly on $\mu_s$, or in other words on the modulation frequency $f_{mod}$ of the laser beam.

One usually estimates that an intensity-modulation frequency $f_{mod}$ of the laser beam of 50 Hz makes it possible to analyze the skin up to a depth of approximately 25 $\mu$m, and that an intensity-modulation frequency $f_{mod}$ of the laser beam of 500 Hz makes it possible to analyze the skin up to a approximately depth of 7 $\mu$m.

In case of skin, as can be seen on Fig. 2, light may meet several layers on its path at these intensity-modulation frequencies : the stratum corneum layer first, then the stratum lucidum layer and next the stratum granulosum layer, as the intensity-modulation frequency decreases.

When in-vivo measurements are intended, the definition of the depth under the skin at which the analyte is measured is however unclear.

First, depending on the pressure exerted on the skin, either by the analysis apparatus or by other objects in contact with the skin (a wristwatch, the armrest of the skin, somebody coming in contact with the patient,etc.)

- the skin surface isn't always at the same distance of the light emitter
- and its 3D-structure can be modified.

Secondly, since the skin is a living tissue, its 3D-structure constantly evolves and the composition and thickness of its different layers can change over time, slow or fast.

A schematic representation of the layers of the skin that can be seen in Fig. 2. This representation is a coarse one. It is only aiming at giving a general idea of the skin structure and does not accurately describe a permanent reality. However, this figure helps understand the impossibility to define accurately the depth at which the skin is analyzed, since neither the skin surface of the skin nor the interfaces between the different layers of the skin can be considered to be flat.

It is as a consequence vain and inaccurate to determine and exploit this depth information in an analysis apparatus.

Furthermore, assuming that the skin can be considered as a homogeneous material, the absorption coefficient $\alpha(\lambda irrad)$ of the skin depends on the patient. For example, it depends on the color of the skin since melanin can absorb the energy of the light beam. So, the relationship between the photoacoustic signal sensed and the analyte concentration is patient-dependent and for a given patient, layer-dependent.

As a consequence, it is impossible to determine a particular modulation frequency $f_{mod}$ which could give reliable blood-glucose measurements at any time for any patient.

To overcome this problem, the invention rather focuses on a method to ensure that, when a measurement is made, the laser beam always (or at least as often as possible) reaches a particular layer of the skin, this layer containing the analyte to be measured, without necessarily measuring its so-called depth. In this way, the measurement can be analyte-specific and its repeatability is improved.

For example, blood glucose is correlated to interstitial glucose. Glucose diffuses from the capillaries of the dermis to the interstitial fluid of the epidermis, which is not vascularized. The interstitial fluid glucose concentration can be as a first approximation considered as constant in a given layer, but it decreases from the dermis towards the skin surface. In particular, there is almost no glucose in the stratum corneum since the stratum corneum contains only dead cells.

To ascertain that the photoacoustic signal sensed is glucose-specific, the light beam must reach a layer under the stratum corneum.

And to make it possible to determine a blood glucose value based on formula 3, one must be able to determine the absorption coefficient $\alpha(\lambda irrad)$, which means one must know which layer is the deepest one reached by the light beam at $\lambda irrad$.

To this purpose, in a process according to the invention, the patient is provided with a portable non-invasive analysis apparatus 1, for example a blood glucose sensor.

Two different measurement modes (or measuring modes) are considered for the analysis apparatus 1 in a current or established operating mode.

In the first mode, or so-called "sweep mode", a frequency sweep is performed one or several times on the intensity-modulation frequency of the laser $f_{mod}$.

The sweep mode acquisitions are in a particular embodiment repeated, for example periodically repeated. The period of the frequency sweep mode acquisitions is called $T_{SM}$.

For example, $T_{SM}$ can be of the order of 30 minutes. This means that every 30 minutes, a frequency sweep is performed and photoacoustic data are acquired in the sweep range.

The sweep rate is chosen slow enough to acquire enough data for further analysis, but fast enough to make it possible to make the subsequent analyte acquisitions required for the monitoring.

For example, the sweep range can be [50 Hz, 50 kHz] or [100 Hz, 15 kHz], and the sweep duration can be of the order of 10 seconds.

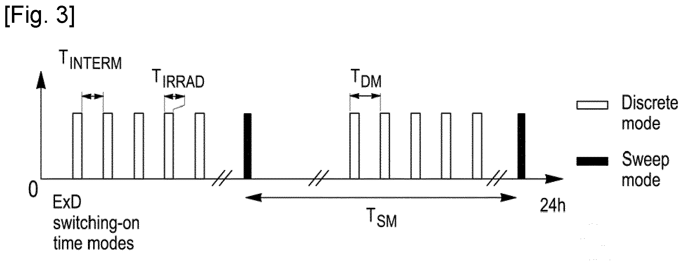

This particular example can be understood in the light of Fig. 3, which is a chronogram showing the switching between the two measuring modes in an exemplary embodiment of the process, but other settings may be convenient, dependent on the use of the analyte sensor.

In the second mode, or so-called "discrete mode", the target is irradiated with a light beam at a given modulation frequency $f_{mod, D.M}$, which is set after at least one sweep mode measurement and based on the results of one ore more of the previous sweep mode acquisition.

The modulation frequency $f_{mod, D.M}$ can remain unchanged until the next sweep mode acquisition.

The discrete mode acquisitions are in a particular embodiment repeated, for example they are periodically repeated. The period of the discrete mode acquisitions is then called $T_{DM}$.

In a particular example, $T_{DM}$ can be of the order of 10 s. This means that a discrete mode acquisition occurs every 10 seconds.

In a particular embodiment, the sweep mode takes priority over the discrete mode in case a sweep mode acquisition should occur at the same time as a discrete mode acquisition.

In this case, one or several discrete mode measurements are suppressed, after what the discrete mode acquisitions resume normally as soon as possible.

Several patterns can be considered for a given discrete mode acquisition. For example, one discrete mode acquisition can comprise the irradiation of the target with the intensity-modulated laser mode, in a pulse mode, during a certain time, called Tirrad.

In another embodiment, one discrete mode acquisition can comprise two successive irradiations of the target with the intensity-modulated laser, in a pulsed or continuous mode, during a certain time, called Tirrad, separated by an intermediate time interval Tinterm during which no light is emitted.

For example, Tirrad is set to one second and Tinterm is set to 5 seconds, which implies that one sweep mode acquisition lasts 7 seconds.

Various patterns can be implemented for a given discrete mode acquisition depending on the precision sought for the analyte measurement and the delay between two analyte measurements.

Repeating the irradiation several times enables to have more accurate estimations of the analyte concentration, since different statistical analysis can be performed, such as calculating a mean-value, can be performed to obtain the whished estimation.

At the same-time, this repeating is time and energy-consuming.

The setting of the number of repetitions, and/or Tirrad and/or Tinterm results for example from a compromise between accuracy and energy and time saving.

After each sweep mode acquisition, the signal processing unit 13 performs a comparison between the data acquired during the last N sweep mode acquisitions, N being a predetermined integer.

The data of the last N sweep mode acquisitions form a time series, containing data in the sweep range.

The time series containing data in the sweep range can be a grouped so as to form a plurality of time series, each of the time series concerning a discrete intensity-modulation frequency in the sweep range or an interval of intensity-modulation frequencies in the sweep range.

N can for example range from 2 up to 1000 acquisitions or more, preferably from 5 to 500, preferably from 5 to 50.

In a particular embodiment, N isn't too high so that the first N-1 acquisitions don't too strongly dominate the results and a change in the Nth acquisition can still be detected.

Several comparisons may be performed on the time series but whatever comparison is performed, since time-series are analyzed, the comparison contains information on the change in the target structure over time.

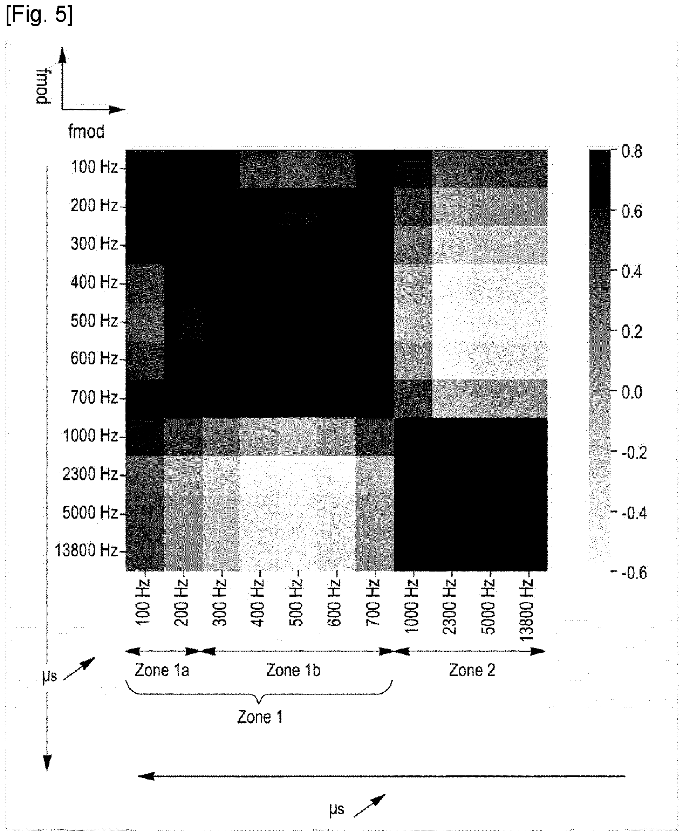

For example, a similarity-dissimilarity-matrix "over time" as shown on Fig. 5 can be calculated for the data acquired for k discrete frequencies in the sweep range.

The acquired data may comprise phase information, and/or amplitude (or intensity) information.

For example, a time series comprising N time points from $t_1$ to $t_N$ would include for each frequency $f_k$ : [($A_k(t_1)$, $\varphi k(t_1)$), ($A_k(t_2)$, $\varphi k(t_2)$),.... ($A_k(t_N)$, $\varphi k(t_N)$)], where $A_k(t_i)$ is the amplitude or the peak-to-peak amplitude of the sensed photoacoustic signal corresponding to an irradiation with a light modulated at $f_{mod} = f_k$.

A similarity-dissimilarity-matrix "over time" is for example a square matrix M(k).

Each coefficient M[i][j] ( for i and j integers in range [1,k]) is obtained by performing a comparison between a time series for the $i^{th}$ frequency and a time series for the $j^{th}$ frequency, each time series being obtained by analyzing the results of the N acquisitions in the sweep mode.

In a particular embodiment, each coefficient M[i][j] ranges from -1 to 1 and is a correlation coefficient between time series i and time series j. A negative coefficient M[i][j] shows an anticorrelation between time series i and time series j, whereas a positive M[i][j] coefficient shows a correlation between time series i and time series j. The anticorrelation or the correlation gets stronger as the absolute value of coefficient M[i][j] increases.

It has been observed in the case of blood glucose that zones of high correlation or high anti-correlation appear in such a similarity-dissimilarity matrix "over time". In other words, the sweep range can be subdivided in several modulation frequencies intervals or zones.

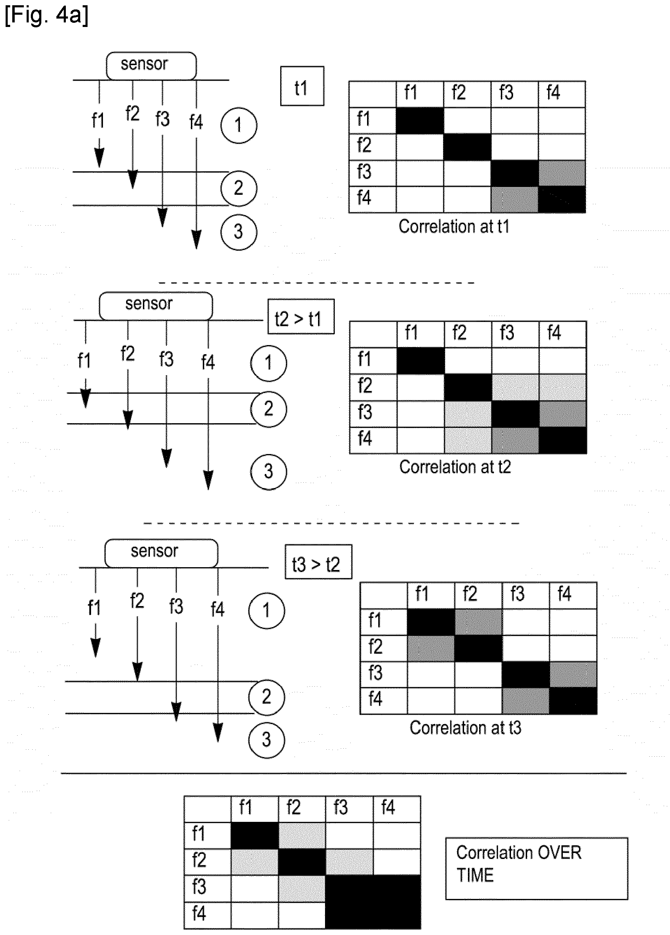

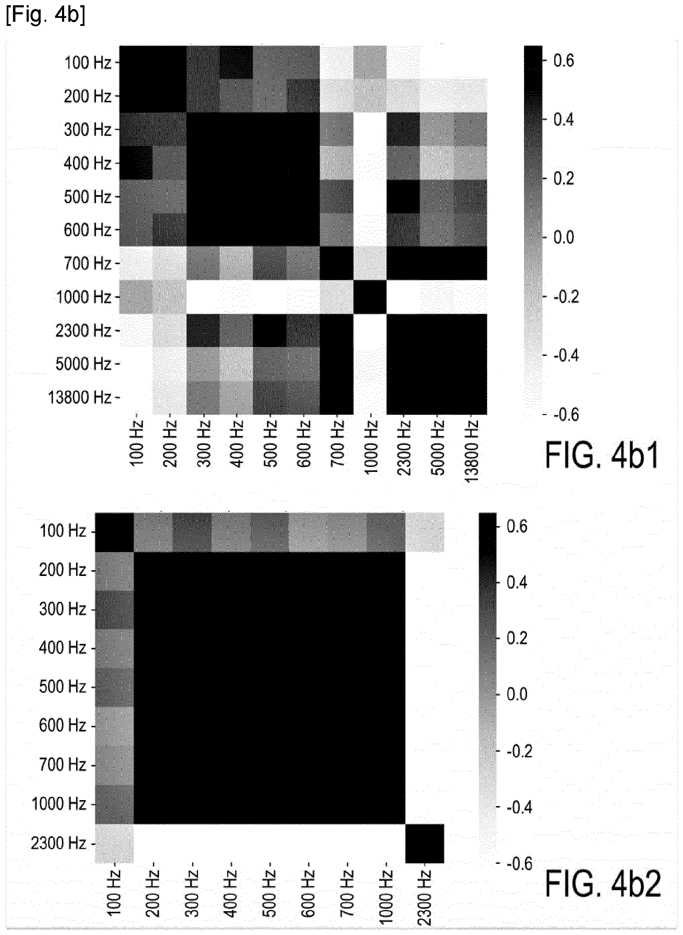

The interest of a similarity-dissimilarity matrix "over time" rather than an "instantaneous" similarity-dissimilarity matrix obtained with an only time point is shown on Fig. 4.

If the skin structure is altered between a first sweep mode acquisition and the next one, the changes will appear in the similarity-dissimilarity matrix "over time" whereas such a change can't be observed in an "instantaneous" similarity-dissimilarity matrix, obtained with an only time point.

To fix one's ideas, let's assume that the targets contain three layers (layer 1, layer 2, layer 3) and that the pressure exerted on the target (or another parameter, such as temperature) changes over time, so that the position of the interfaces between two successive layers change.

In the example of fig. 4a, the pressure P is higher at an instant t2 than at 11, greater at t2 than at t3, and greater at t1 than at t3 : P(t2) > P(t1) > P(t3) and t1 < t2 < t3.

Let's also consider as a first approximation that a particular light intensity-modulated at a given intensity-modulation frequency reaches always a material at a given distance from the light emitter of the sensor.

For the first sweep mode acquisition at t1, intensity-modulation frequency f1 allows to reach the skin just above the first interface layer 1/ layer 2, and intensity-modulation frequency f2 allows to reach the skin just below this first interface layer 1/ layer 2. f1 and f2 aren't instantaneously correlated.

Due to the increased pressure, the interface moves closer to the light emitter for the second acquisition. Consequently, intensity-modulation frequency f1 allows to reach the skin under the first interface layer 1/ layer 2, and intensity-modulation frequency f2 allows to reach the skin under a second interface layer 2/ layer 3. f1 and f2 aren't still instantaneously correlated.

At t3 on the contrary, f1 and f2 both reach layer 1 so they are instantaneously correlated.

The same analysis leads to conclude that f3 and f4 are always instantaneously correlated since they always reach the same layer, layer 3.

In this case, one can see on the correlation matrix "over time" that the time series for f1 and f2 will have a low correlation, which indicates that one must avoid these frequencies in order to have repeatable measurements : f1 and f2 do not reach always the same layer at different times. On the contrary, the time series for f3 and f4 are strongly correlated, indicating that a given layer is always reached by either f3 or f4 or any frequency between f3 and f4.

As a general conclusion, time series at several successive frequencies that are mutually highly correlated indicate that a given skin layer is reached repeatably by a light beam modulated at one of these frequencies. Time series at several successive frequencies that are poorly correlated indicate that an interface, prone to frequent depth variations, is reached by a light beam modulated at one of these frequencies.

The core of a layer can change in depth but it will always be in a given layer, whereas in the vicinity of an interface, the tissue can sometimes be associated with the upper layer, sometimes with the lower layer, depending on the pressure, the temperature,..., exerted on the skin. Choosing a modulation frequency between several successive frequencies that are mutually highly correlated ensures that the core of a layer is sensed, even if the width of the layer fluctuates moderately.

As a consequence, performing several sweep mode acquisitions in order to compare the time series over time in the sweep range and adapting in consequence the settings of the sensor for a subsequent analyte measurement helps address the problem of measurement repeatability.

The number N of first mode acquisitions to be performed depends on the characteristic times of the changes in the target structure that are of importance for subsequent analysis.

Fig. 4b shows the results of an experiment carried out on an anesthetized pig, with 106 first mode irradiations, each irradiation lasting one minute, the whole experiment lasting around two hours. Fig 4b,1 shows the similarity-dissimilarity matrix "overtime" for the first 53 time points, and Fig 4b,2 shows the similarity-dissimilarity matrix "over time" for the last 53 time points.

It appears clearly that the frequencies in the range [300 Hz, 600 Hz] are correlated for the 106 acquisitions, as well as for the first 53 or for the last 53 acquisitions. On the contrary, the frequencies in the range [700, 1000 Hz] are poorly correlated with one another and with the frequencies in the range [300 Hz, 600 Hz] for the first 53 whereas they are highly correlated during the last 53 acquisitions.

If the frequencies in the range [700, 1000 Hz] are considered to be of interest, the first mode acquisitions need to be more frequent than if frequencies in the range [300 Hz, 600 Hz] are selected for subsequent measurement.

It also appears clearly that it is impossible to obtain such information with an instantaneous correlation matrix.

In a particular embodiment, the predetermined integer N can be different in an initialization phase and in an established mode, or can be adapted during the established mode for example based on the results of the preceding analysis.

In order to choose an intensity-modulation frequency $f_{mod,D.M}$ for an analyte measurement, several strategies can be foreseen.

In one embodiment, one or more correlation threshold can for example be first predetermined.

A correlation threshold can take into account the results of a former learning phase and/or models.

One or more zones can then be identified, based on the following definition : any pair of times series within the frequency range of a frequency zone has a correlation coefficient greater than the predetermined correlation threshold.

Depending on the value of the correlation threshold, in the example of Fig. 5, at three zones (Zone 1a, Zone 1b and Zone 2) in which the time series are strongly correlated can be identified.

The determination of one or more frequency zones can also be based on one or more anticorrelation threshold.

Fig. 5 shows the results of an experiment carried out on an anesthetized pig, with 98 first mode irradiations, each irradiation lasting one minute, the whole experiment lasting around two hours.

In the example of Fig.5, Zone 1b and Zone 2 are strongly anti-correlated whereas no anti-correlation is observed between Zone 1a and Zone 1b. Taking into account both an anticorrelation threshold and a correlation threshold, the first zone (Zone 1) may as a consequence not be subdivided into the two subzones (Zone 1a and Zone 1b)

Keeping in mind that increasing the modulation frequency $f_{mod}$ means probing at a shallower depth, one can deduce that there is at least one interface between two skin layers, and this interface is reached for intensity-modulation frequency around 1 kHz. With a modulation frequency $f_{mod}$ ranging in Zone 2, namely ] 1 kHz, 13,8 kHz], a first layer, just beneath the skin surface is analyzed, whereas with a modulation frequency $f_{mod}$ ranging in Zone 1, namely [50 Hz, 1 kHz[, a second layer, below the first layer, is analyzed.

In addition, one should avoid the border between the two zones to ensure repeatability.

At this step, one knows that intensity-modulation frequency $f_{mod, D.M}$ for an analyte measurement, can be chosen far from 1kHz, either in zone 1 or in zone 2.

One purpose of the process is to allow such an identification of the different layers in the target 2, at least by counting the layers, and possibly analyzing the content of these layers. One can for example make use of this information in another device, such as a drug delivery device.

In another embodiment, one ore more correlation threshold can be determined based on a predetermined criteria. One can for example assume that three or four zones are expected in the similarity-dissimilarity model.

Based on a skin model, it could thus be decided that three different zones should always be found. In this case, the one or more thresholds could be calculated in order to ensure that these three zones are identified. These thresholds would as a consequence be mobile between two decisions on the intensity-modulation frequency $f_{mod, D.M}$.

In this case, one could further set a predetermined lower threshold in order to ensure that the zones aren't related to noise but to an analyte-specific signal.

If the first step consists in identifying one or more zones in the target, a next step would be the choice of one of the zones for a subsequent analyte measurement.

In the case of skin, the target is a multilayered materiel. In first approximation each layer k can be associated with a particular absorption coefficient $\alpha(\lambda irrad, k)$. In a particular embodiment, p successive layers are detected based on the frequency zones identified in the similarity-dissimilarity matrix "over time".

In the example of Fig. 5, two layers are detected. The first (shallowest) layer, corresponding to Zone 2, is probably the stratum corneum. Considering the large frequency range of the second zone, the second (deepest) layer, can possibly comprises the stratum lucidum, the stratum granulosum, and part of the stratum spinosum, which have similar content of water and/or glucose. The second layer could then be subdivided in two sub-layers if needed.

As explained earlier, in the case of a blood glucose sensor, the stratum corneum is of no interest for an interstitial glucose measurement. To ensure that a subsequent is a glucose-specific measurement, one can choose an intensity-modulation frequency in Zone 1 for this subsequent measurement.

In another embodiment, the determination of the modulation frequency of interest can be based on another multilayer-model or other optimization criteria.

One could for example consider that there is a gradient of interstitial glucose concentration, decreasing from the dermis to the epidermis as we move away from the capillaries.

Regardless of the skin model, we know that there is a delay between blood glucose and interstitial glucose and that this delay is all the greater as the measurement is performed far from the capillaries of the dermis.

If interstitial glucose is measured at time t, it is known to be correlated with blood glucose at time t'< t but t'-t depends on where the measurement is performed.

The process according to the invention allows to address this problem in so far at it ensures that the measurement is as often as possible performed at approximately the same location, that is to say at least the same layer of the skin.

The determination of the modulation frequency can also include a learning phase.

One understands that it is possible to set an intensity-modulation frequency for a subsequent measurement without further information on the skin structure.

In other words, in any embodiment of a glucose sensor, after the calculation of the similarity-dissimilarity matrix "over time", a modulation frequency $f_{mod, D.M}$ may be set, that ensures that a particular layer deeper than the stratum corneum is reached, this choice being made without prior knowledge of the skin structure of a given patient and without resorting to a depth parameter, that we are unable to define accurately.

Again in other words, the similarity-dissimilarity matrix "over time" helps ensuring that the sensor signal is analyte-specific for a given measurement and it improves the reproducibility of the measurements.

In one embodiment, to choose a particular modulation frequency $f_{mod, D.M}$, it can be decided that $f_{mod, D.M}$ is in mathematical relation with the frequency interval characterizing a particular zone in the similarity-dissimilarity matrix "over time".

For example, $f_{mod, D.M}$ can be the mean frequency of this interval so as to be as far as possible from the interfaces with other target zones.

Or one could take into account that the thermal diffusion length ($\mu_s$) isn't linearly related to the intensity-modulation frequency.

In another embodiment, the measurement sensitivity can be optimized, once the measurement repeatability has been addressed.

In an exemplary embodiment, the largest frequency zone (or frequency interval) identified in the matrix that is also comprised in [50 Hz, 700 Hz] is selected to ascertain sufficient sensitivity in case of a glucose sensor. Then, the lowest frequency of this selected frequency zone (Zone 1b, 200 Hz in the example of Fig. 5) is chosen for $f_{mod, D.M}$ to improve the measurement sensitivity, since it has been observed that a lower $f_{mod, D.M}$ is associated with a stronger signal, all other things being equal.

Once $f_{mod, D.M}$ has been set, it is kept constant during P discrete mode acquisitions, until the next sweep mode acquisition occurs after a $T_{SM}$ time lapse. In our exemplary embodiment, $f_{mod, D.M}$ is kept constant during 30 minutes.

$T_{SM}$ after the last sweep mode acquisition, a new sweep-mode acquisition is performed. The acquired data are added to the time series.

A new similarity-dissimilarity matrix "over time" is calculated with the N latest points of the time series. After calculating this new similarity-dissimilarity matrix "over time", a particular modulation frequency $f_{mod, D.M}$ is fixed. This particular modulation frequency $f_{mod, D.M}$ ensures that a particular layer is reached (or a particular layer is avoided if needed), this choice being made without knowing the target structure, for example the skin structure of a particular patient, and without resorting to a depth parameter, that we are unable to define accurately. The intensity-modulation frequency $f_{mod, D.M}$ is once again kept constant for the next P discrete mode acquisitions, and so on.

P can be equal to 1 but since energy-consumption is higher in the sweep-mode than in the discrete mode, it may be preferable that P is more than 1, for example 5, 10 or more.

$T_{SM}$ is long enough to perform P discrete mode acquisitions between two successive sweep mode acquisitions. But it is at the same time short enough to ensure the repeatability of the measurement : if $T_{SM}$ is too long, middle term variations in the structure of the skin that can alter the measurements won't be corrected by a regular adaptation of intensity-modulation frequency $f_{mod,D.M}$.

Of course, having data over N sweep mode acquisitions implies an initialization phase.

For example, during the initialization phase, $f_{mod,D.M}$ is set at a default value and no comparison is performed after the sweep mode acquisition until N is reached.

In another embodiment, during the initialization phase, N successive sweep mode acquisitions are performed separated by an initialization period Tinit, Tinit being for example shorter, or even much shorter, than $T_{SM}$ in the established operating mode.

Since this sweep mode requires little time and little energy after the initialization phase, it is possible to consider periodic sweep mode acquisitions.

After each sweep mode acquisition in the established operating mode, a patient-specific and environment-specific $f_{mod,D.M}$ is set : $f_{mod,D.M}$ can be patient-specific since the data used to calculate the similarity-dissimilarity matrix "over time" can all be acquired on a given patient in an exemplary embodiment; environment-specific since the data used to calculate the similarity-dissimilarity matrix "over time" result from given conditions at a given period of time that immediately precedes the next discrete mode measurements.

Consequently, the process improves the accuracy and the repeatability of the photoacoustic detection of an analyte. This improvement is obtained at reasonable time and energy costs.

Other comparison techniques may be used to calculate the similarity-dissimilarity matrix "over time". For example, Dyamic Time Warping calculations may be performed.

In a particular embodiment, each coefficient M[i][j] of the similarity-dissimilarity matrix "over time" ranges from 0 to 100 and is the Dynamic Time Warping Distance (DTW-Distance) between time series i and time series j. A low coefficient M[i][j] shows a low DTW-distance between time series i and time series j, and as a consequence a high similarity between these time series. In this case, the similarity between time series i and time series j decreases as coefficient M[i][j], or equivalently DTW-Distance, increases.

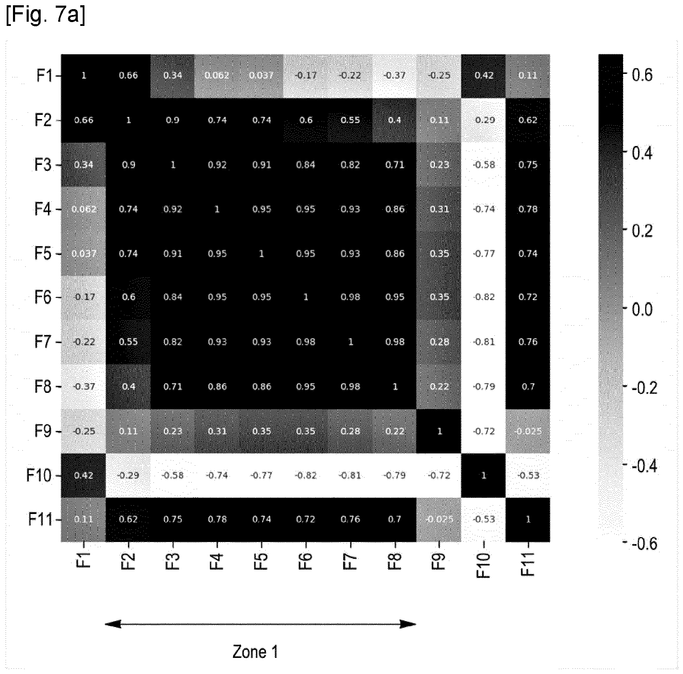

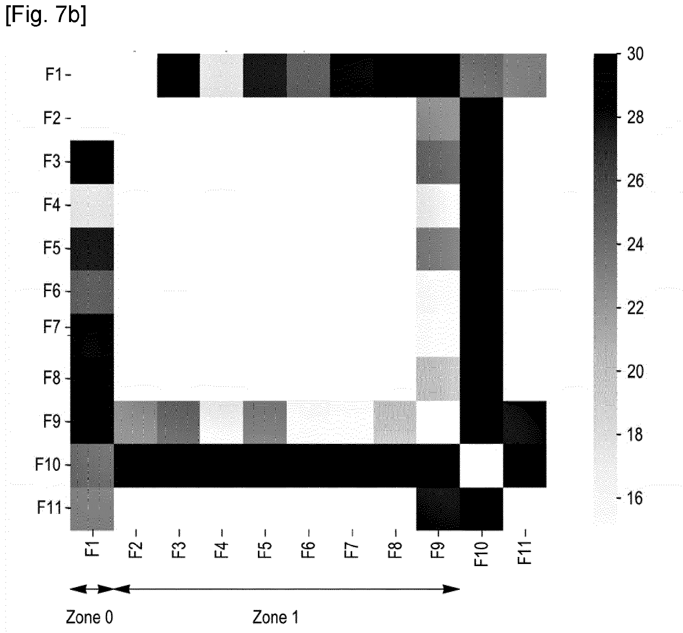

An example of such a DTW matrix "over time" can be seen in Fig. 7b, the correlation matrix "over time" being presented on Fig. 7a.

Fig. 7a and 7b show the results of an experiment carried out on an anesthetized pig, with 130 first mode irradiations, each irradiation lasting one minute, the whole experiment lasting around two hours, the modulation frequency being swept from 100 Hz up to 2.3 kHz.

In the example of Fig. 7a and Fig. 7b, the DTW analysis or the correlation analysis lead to similar conclusions for Zone 1, but the DTW analysis could lead to the conclusion that a zone 0 can be identified corresponding to frequencies f1 and f2, since the DTW distance between f1 and f3 is very high whereas the distance between f1 and f2 is very low.

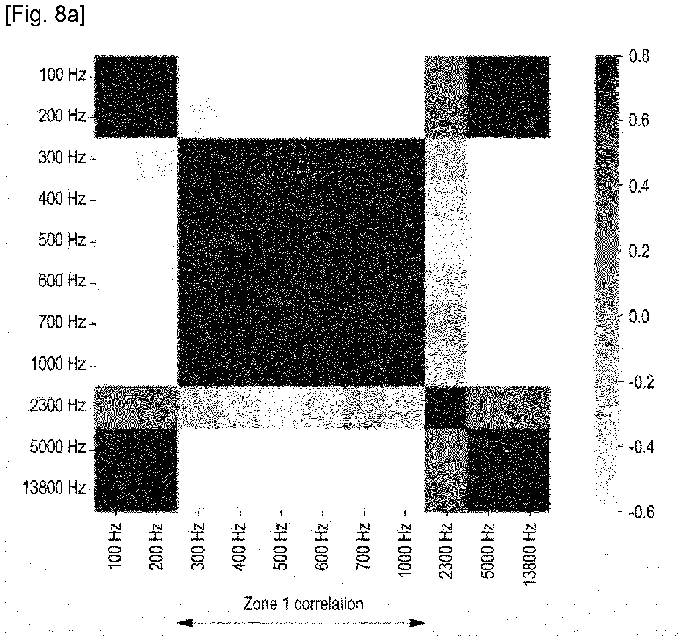

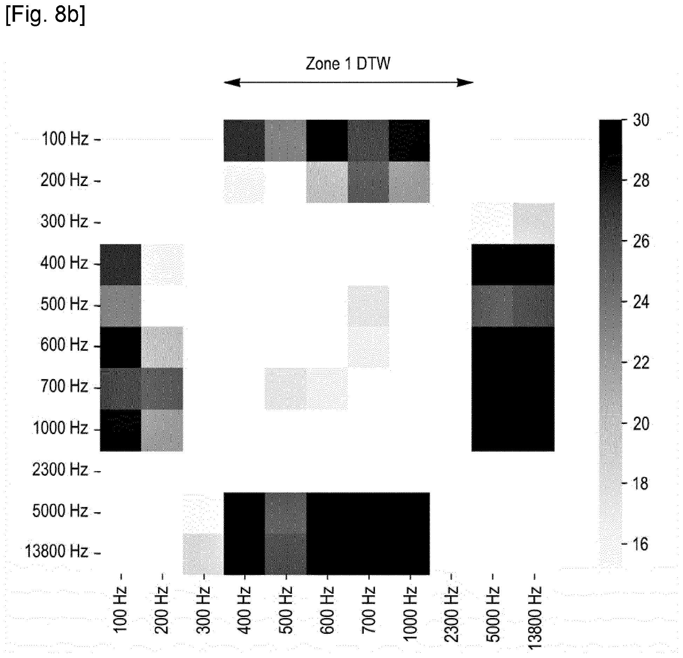

Fig. 8a and 8b show the results of an experiment carried out on an anesthetized pig, with 60 first mode irradiations, each irradiation lasting one minute, the whole experiment lasting around two hours, the modulation frequency being swept from 100 Hz up to 13.8 kHz.

In this case, the correlation analysis would lead to the conclusion that a zone 1 corresponds to modulation frequencies ranging from 300 Hz to 1000 Hz, whereas the DTW analysis would lead to the conclusion that zone 1 corresponds to modulation frequencies ranging from 400 Hz to 2300 Hz

One understands that depending for example on the analyte, the patient or the precision sought, one of theses two comparison techniques, or another comparison technique, or several comparison techniques can be implemented.

That is to say, two comparison techniques can give different, and sometimes complementary, insights into the structure of the skin over time.

For example, DTW calculations may provide information on a change in the delay between blood glucose and interstitial blood glucose.

In a particular embodiment, $f_{mod,D.M}$ is set based on the results of different comparison techniques.

One can note that a photoacoustic detection cell may be of particular interest in the case of blood glucose monitoring : first because the data acquired contain both an amplitude (or intensity) and a phase information, second because an only light emitter is required, which reduces the energy consumption compared to detection techniques involving two lasers. As a consequence, the autonomy of a portable blood glucose sensor on which a process according to the invention is implemented can be increased.

Additional information can be inferred from the similarity-dissimilarity matrix "over time".

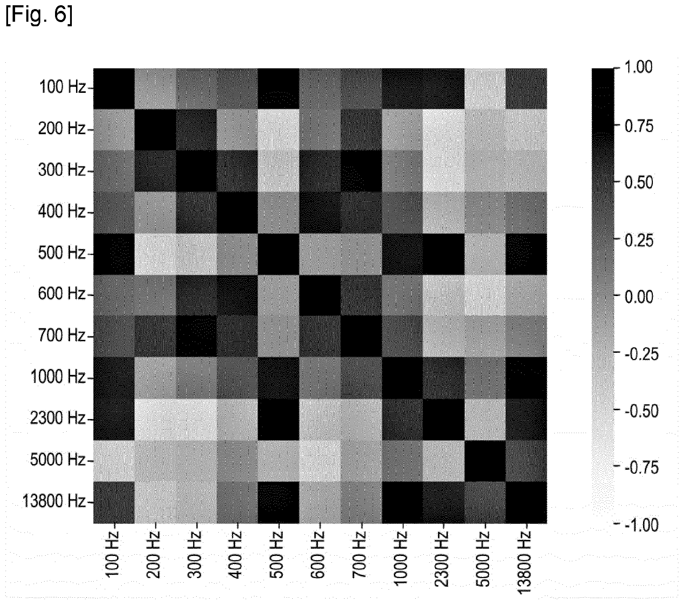

In the example of Fig. 6, no particular correlation or anti-correlation zone can be identified in the similarity-dissimilarity matrix "over time", whereas there should have been some, since the data acquired in a particular layer, corresponding to a particular modulation frequency interval in the sweep range should be correlated.

Note that it is very unlikely to obtain an "instantaneous" similarity-dissimilarity matrix such as the one Fig. 6 since at a given time, whereas if there is a persisting problem with the sensor or the patient, dissimilarities will accumulate over time and appear very clearly only over time.

This indicates that problems have occurred during the repeated sweep-mode acquisitions. Either the analyte sensor 1 is defective or not correctly positioned on the patient's skin.

Whatever the explanation, an alarm may for example be triggered to alert the user that a analyte sensor 1 must be checked.

In case an alarm has been raised, once the analyte sensor 1 has been checked, a new initialization phase can be started in order to acquire a time series containing the data corresponding to N sweep-mode acquisitions, all of them consistent with for example a modified location or tightening of the analysis apparatus 1.

Or if the alarm was raised for other technical reasons that allows to consider some of the preceding sweep-mode acquisitions are still consistent with a sweep-mode acquisition performed once the analysis apparatus 1 has been checked, a partial initialization phase can be started to complete the time series up to N points, or if not needed, the sensor can resume the established operating mode.

As said above, the frequency sweep mode acquisitions period $T_{SM}$ can be greater than the discrete mode acquisitions period $T_{DM}$ For example, $T_{SM}$ is long enough to have sustainable power consumption (since power consumption is particularly high in the sweep mode) but short enough to guarantee with a high confidence level that all the discrete mode acquisitions between two sweep-mode acquisitions are consistent.

In one embodiment, a first sensor is provided in the detection cell for carrying out steps a and b of the process and a second sensor is provided for carrying out step e of the process.

In this manner, one sensor can be dedicated to the first mode acquisitions and another one to the second mode acquisition. It is even possible to use different type of sensors or the same type of sensors but with different performance for the first mode acquisitions and the second mode acquisitions, depending for example on the accuracy required for each mode.

A sensor can for example be a thermal sensor or an electroacoustic sensor.

Preferably, but not mandatorily, both sensors are located close to each another.

The possibilities of miniaturization and of integration in an integrated system are also taken into account.

In one embodiment, in the process for non-invasively analyzing a target with an analysis apparatus, a second light emitter emitting an intensity-modulated light at a controllable modulation frequency is provided for carrying the at least one irradiation at step e.

In this manner, one light emitter can be dedicated to the first mode acquisitions and another one to the second mode acquisitions. It is even possible to use different type of light emitters for the first mode acquisitions and the second mode acquisitions, depending for example on the power or the quality of beam required for each mode.

Preferably, but not mandatorily, both light emitters are located close to each another, so that the analysis both light beams reach approximately the same zone of the target.

An integrated system for monitoring an analyte concentration in a host and for delivering a medicament to a host can be provided, the system comprising a continuous analyte sensor 1 implementing a process according to the invention. The analyte values calculated by the processor module of the analyte sensor 1 can then be used to determine, once or iteratively, a medicament therapy.

## LIST OF THE REFERENCE SIGNS

1 : portable non-invasive analysis apparatus
11 : light emitter block
12 : detection cell
13 : signal processing module
2 : target

## Claims

1. Process for non-invasively analyzing a target with an analysis apparatus (1) comprising:

- an intensity-modulation device, a light emitter emitting an intensity-modulated light at a controllable modulation frequency,
- at least one detection cell (12) comprising a sensor sensing directly or indirectly a thermal wave propagating out of the target in response to an irradiation of said target by the light emitted,
- a processor module (13) configured to receive and process sensor data from at least one detection cell, wherein the process comprises:

   a. carrying out at least one irradiation of the target with the light emitter in a first mode, the modulation frequency of the intensity of the light emitted being swept during each of the at least one irradiation in the first mode over a frequency sweep range, and the processor module receiving sensor data from a detection cell for each of the at least one irradiation in the first mode,

b. carrying out at least one additional irradiation of said target with said light emitter in said first mode, the processor module receiving sensor data from a detection cell for each of the at least one additional irradiation in the first mode,

c. processing with the processor module (13) the sensor data received for at least two irradiations in said first mode and forming with the processor module (13) a time series comprising sensor data for each of a plurality of discrete frequencies in the frequency sweep range,

d. calculating, using the processor module (13), a discrete mode modulation frequency based on an analysis of the time series comprising sensor data for each of a plurality of discrete frequencies in the frequency sweep range, said analysis comprising forming a time series for each of a plurality of discrete frequencies and calculating at least one value indicative of the similarity or the dissimilarity between two time series at two of the plurality of discrete frequencies,

e. carrying out at least one irradiation of the target with a light emitter emitting an intensity-modulated light in a second mode, the modulation frequency of the intensity of the light emitted in the second mode being said discrete mode modulation frequency calculated at step d, the processor module (13) receiving sensor data from a detection cell for each of the at least one irradiation in the second mode,

f. calculating, using the processor module (13), an analyte value based on the sensor data received for at least one of the irradiation in the second mode.

2. Process for non-invasively analyzing a target with an analysis apparatus (1) according to claim 1 **characterized in that** a first sensor is provided for carrying out steps a and b and a second sensor is provided for carrying out step e.

3. Process for non-invasively analyzing a target with an analysis apparatus (1) to claim 1 or claim 2 **characterized in that** a second light emitter emitting an intensity-modulated light at a controllable modulation frequency is provided for carrying the at least one irradiation at step e.

4. Process for non-invasively analyzing a target with an analysis apparatus (1) according to any of claims 1 to 3 **characterized in that** at step e, at least two irradiations in the second mode are performed, two successive irradiations in the second mode being separated by a first period of time TDM.

5. Process for non-invasively analyzing a target with an analysis apparatus (1) according to claim 4 **characterized in that** at step f, the analyte value is calculated based on the sensor data received for a plurality of the at least two irradiations in the second mode at the preceding step e.

6. Process for non-invasively analyzing a target with an analysis apparatus (1) according to any of claims 1 to 5 **characterized in that** steps b, c, d, and e are periodically repeated in this order, two successive steps b being separated by a second period of time TSM.

7. Process for non-invasively analyzing a target with an analysis apparatus (1) according to any of claims 4 -5 and to claim 6 **characterized in that** the first period of time TDM is shorter than said second period of time TSM.

8. Process for non-invasively analyzing a target with an analysis apparatus (1) according to any of claims 6 or 7 **characterized in that** a first integer N is predetermined and that at step c, the time series is formed with the data acquired during the N latest irradiations in the first mode.

9. Process for non-invasively analyzing a target with an analysis apparatus (1) according to claim 8 **characterized in that** at step a, N-1 irradiations are performed in the first mode, two successive irradiations of step a being separated by a third period of time TDM.

10. Process for non-invasively analyzing a target with an analysis apparatus (1) according to any of claims 1 to 9 **characterized in that** at step d, the analysis of the time series comprising sensor data for each of a plurality of discrete frequencies in the frequency sweep range further comprises performing a dynamic time warping algorithm and/or a correlation analysis on this time series.

11. Process for non-invasively analyzing a target with an analysis apparatus (1) according to any of claims 1 to 10 **characterized in that** an intensity-modulated light comprises at least one wavelength that is absorbed by an analyte to be measured in the target.

12. Process for non-invasively analyzing a target with an analysis apparatus (1) according to claim 11 **characterized**

**in that** the at least one wavelength is within the mid infrared range.

13. Process for non-invasively analyzing a target with an analysis apparatus (1) according to any of claims 1 to 12 **characterized in that** the analysis apparatus further comprises a user interface or is further configured to transmit data to a medicament delivery device, and **characterized in that** at step d, the results of the analysis of the time series comprising sensor data for each of a plurality of discrete frequencies in the frequency sweep range are compared to a predetermined threshold, and **in that**, based on the result of this comparison, an alarm signal is displayed on the user interface or transmitted to the medicament delivery device.

14. Process for non-invasively analyzing a target with an analysis apparatus (1) according to any of claims 1 to 13 **characterized in that** at least one detection cell comprises a sensor chosen in the list {electroacoustic sensor, thermal sensor}.

15. Apparatus (1) for non-invasively analyzing a target comprising:

- at least one intensity-modulation device, a light emitter emitting an intensity-modulated light at a controllable modulation frequency,
- at least one detection cell (12) comprising a sensor sensing directly or indirectly a thermal wave propagating out of the target in response to an irradiation of said target by the light emitted,
- at least one processor module (13) configured to receive and process sensor data from at least one detection cell, wherein at least one light emitter is configured to:

a. carry out at least one irradiation of the target with the light emitter in a first mode, the modulation frequency of the intensity of the light emitted being swept during each of the at least one irradiation in the first mode over a frequency sweep range,
b. carry out at least one additional irradiation of said target with said light emitter in said first mode, wherein the at least one processor module (13) is configured to
c. receive sensor data from at least one detection cell for each of the at least one irradiation in a first mode,
d. process the sensor data received for at least two irradiations in said first mode and form a time series comprising sensor data for each of a plurality of discrete frequencies in the frequency sweep range, and
e. calculate a discrete mode modulation frequency based on an analysis of the time series comprising data for each of a plurality of discrete frequencies in the frequency sweep range, said analysis comprising forming a time series for each of a plurality of discrete frequencies and calculating at least one value indicative of the similarity or the dissimilarity between two time series at two of the plurality of discrete frequencies,

and wherein :

- the at least one light emitter is configured to carry out at least one irradiation of the target with the light emitter in a second mode, the light emitted in the second mode being intensity-modulated at said discrete mode modulation frequency calculated by at least one processor module, and
- the at least one processor module is configured to receive sensor data from at least one detection cell for each of the at least one irradiation in the second mode and to calculate an analyte value based on the sensor data received for at least one irradiation in the second mode.

16. Apparatus (1) for non-invasively analyzing a target according to claim 15 **characterized in that** the apparatus is wearable.

17. System comprising an apparatus (1) for non-invasively analyzing a target according to any of claims 15 to 16 and a medicament delivery device, the apparatus for non-invasively analyzing a target being further configured to send data to the medicament delivery device and the system being further configured to determine a medicament therapy to be delivered by the medicament delivery device based on at least one analyte value calculated with the processor module of the apparatus for non-invasively analyzing a target.

**Patentansprüche**

1. Verfahren zum nicht-invasiven Analysieren eines Targets mit einem Analysegerät (1), das Folgendes umfasst:

- eine Intensitätsmodulationsvorrichtung, einen Lichtemitter, der ein intensitätsmoduliertes Licht mit einer regelbaren Modulationsfrequenz emittiert,
- mindestens eine Detektionszelle (12), die einen Sensor umfasst, der direkt oder indirekt eine sich aus dem Target ausbreitende Wärmewelle als Reaktion auf eine Bestrahlung des genannten Targets durch das emittierte Licht erfasst,
- ein Prozessormodul (13), das zum Empfangen und Verarbeiten von Sensordaten von mindestens einer Detektionszelle konfiguriert ist, wobei das Verfahren Folgendes umfasst:

a. Durchführen mindestens einer Bestrahlung des Targets mit dem Lichtemitter in einem ersten Modus, wobei die Modulationsfrequenz der Intensität des emittierten Lichts während jeder der mindestens einen Bestrahlung im ersten Modus über einen Frequenzdurchlaufbereich durchlaufen wird und das Prozessormodul Sensordaten von einer Detektionszelle für jede der mindestens einen Bestrahlung im ersten Modus empfängt,

b. Durchführen mindestens einer zusätzlichen Bestrahlung des genannten Targets mit dem genannten Lichtemitter in dem genannten ersten Modus, wobei das Prozessormodul Sensordaten von einer Detektionszelle für jede der mindestens einen zusätzlichen Bestrahlung im ersten Modus empfängt,

c. Verarbeiten, mit dem Prozessormodul (13), der für mindestens zwei Bestrahlungen in dem genannten ersten Modus empfangenen Sensordaten und Bilden, mit dem Prozessormodul (13), einer Zeitreihe, die Sensordaten für jede einer Vielzahl von diskreten Frequenzen im Frequenzdurchlaufbereich umfasst,

d. Berechnen, mit Hilfe des Prozessormoduls (13), einer Diskretmodus-Modulationsfrequenz auf der Basis einer Analyse der Zeitreihe, die Sensordaten für jede einer Vielzahl von diskreten Frequenzen im Frequenzdurchlaufbereich umfasst, wobei die genannte Analyse das Bilden einer Zeitreihe für jede einer Vielzahl von diskreten Frequenzen und das Berechnen mindestens eines Wertes umfasst, der die Ähnlichkeit oder die Unähnlichkeit zwischen zwei Zeitreihen bei zwei der Vielzahl von diskreten Frequenzen angibt,

e. Durchführen mindestens einer Bestrahlung des Targets mit einem Lichtemitter, der ein intensitätsmoduliertes Licht in einem zweiten Modus emittiert, wobei die Modulationsfrequenz der Intensität des im zweiten Modus emittierten Lichts die genannte in Schritt d berechnete Diskretmodus-Modulationsfrequenz ist, wobei das Prozessormodul (13) Sensordaten von einer Detektionszelle für jede der mindestens einen Bestrahlung im zweiten Modus empfängt,

f. Berechnen, mit Hilfe des Prozessormoduls (13), eines Analytwerts auf der Basis der für mindestens eine der Bestrahlungen im zweiten Modus empfangenen Sensordaten.

2. Verfahren zum nicht-invasiven Analysieren eines Targets mit einem Analysegerät (1) nach Anspruch 1, **dadurch gekennzeichnet, dass** ein erster Sensor zum Durchführen der Schritte a und b bereitgestellt wird und ein zweiter Sensor zum Durchführen von Schritt e bereitgestellt wird.

3. Verfahren zum nicht-invasiven Analysieren eines Targets mit einem Analysegerät (1) nach Anspruch 1 oder Anspruch 2, **dadurch gekennzeichnet, dass** ein zweiter Lichtemitter, der ein intensitätsmoduliertes Licht mit einer regelbaren Modulationsfrequenz emittiert, zum Durchführen der mindestens einen Bestrahlung in Schritt e bereitgestellt wird.

4. Verfahren zum nicht-invasiven Analysieren eines Targets mit einem Analysegerät (1) nach einem der Ansprüche 1 bis 3, **dadurch gekennzeichnet, dass** in Schritt e mindestens zwei Bestrahlungen im zweiten Modus durchgeführt werden, wobei zwei aufeinanderfolgende Bestrahlungen im zweiten Modus durch eine erste Zeitspanne TDM getrennt sind.

5. Verfahren zum nicht-invasiven Analysieren eines Targets mit einem Analysegerät (1) nach Anspruch 4, **dadurch gekennzeichnet, dass** in Schritt f der Analytwert auf der Basis der für eine Vielzahl der mindestens zwei Bestrahlungen im zweiten Modus im vorhergehenden Schritt e empfangenen Sensordaten berechnet wird.

6. Verfahren zum nicht-invasiven Analysieren eines Targets mit einem Analysegerät (1) nach einem der Ansprüche 1 bis 5, **dadurch gekennzeichnet, dass** die Schritte b, c, d und e periodisch in dieser Reihenfolge wiederholt werden, wobei zwei aufeinanderfolgende Schritte b durch eine zweite Zeitspanne TSM getrennt sind.

7. Verfahren zum nicht-invasiven Analysieren eines Targets mit einem Analysegerät (1) nach einem der Ansprüche 4-5 und nach Anspruch 6, **dadurch gekennzeichnet, dass** die erste Zeitspanne TDM kürzer ist als die genannte zweite Zeitspanne TSM.

8. Verfahren zum nicht-invasiven Analysieren eines Targets mit einem Analysegerät (1) nach Anspruch 6 oder 7, **dadurch gekennzeichnet, dass** eine erste ganze Zahl N vorgegeben wird und dass in Schritt c die Zeitreihe mit den während der N letzten Bestrahlungen im ersten Modus erfassten Daten gebildet wird.

9. Verfahren zum nicht-invasiven Analysieren eines Targets mit einem Analysegerät (1) nach Anspruch 8, **dadurch gekennzeichnet, dass** in Schritt a N-l Bestrahlungen im ersten Modus durchgeführt werden, wobei zwei aufeinanderfolgende Bestrahlungen von Schritt a durch eine dritte Zeitspanne TDM getrennt sind.

10. Verfahren zum nicht-invasiven Analysieren eines Targets mit einem Analysegerät (1) nach einem der Ansprüche 1 bis 9, **dadurch gekennzeichnet, dass** in Schritt d die Analyse der Zeitreihe, die Sensordaten für jede einer Vielzahl von diskreten Frequenzen im Frequenzdurchlaufbereich umfasst, ferner das Durchführen eines dynamischen Zeitverzerrungsalgorithmus und/oder einer Korrelationsanalyse an dieser Zeitreihe umfasst.

11. Verfahren zum nicht-invasiven Analysieren eines Targets mit einem Analysegerät (1) nach einem der Ansprüche 1 bis 10, **dadurch gekennzeichnet, dass** ein intensitätsmoduliertes Licht mindestens eine Wellenlänge umfasst, die von einem zu messenden Analyten im Target absorbiert wird.

12. Verfahren zum nicht-invasiven Analysieren eines Targets mit einem Analysegerät (1) nach Anspruch 11, **dadurch gekennzeichnet, dass** die mindestens eine Wellenlänge im mittleren Infrarotbereich liegt.

13. Verfahren zum nicht-invasiven Analysieren eines Targets mit einem Analysegerät (1) nach einem der Ansprüche 1 bis 12, **dadurch gekennzeichnet, dass** das Analysegerät ferner eine Benutzeroberfläche umfasst oder ferner zum Übertragen von Daten zu einer Medikamentenabgabevorrichtung konfiguriert ist, und **dadurch gekennzeichnet, dass** in Schritt d die Ergebnisse der Analyse der Zeitreihe, die Sensordaten für jede einer Vielzahl von diskreten Frequenzen im Frequenzdurchlaufbereich umfasst, mit einem vorbestimmten Schwellenwert verglichen werden, und dadurch, dass auf der Basis des Ergebnisses dieses Vergleichs ein Alarmsignal auf der Benutzeroberfläche angezeigt oder zu der Medikamentenabgabevorrichtung übertragen wird.

14. Verfahren zum nicht-invasiven Analysieren eines Targets mit einem Analysegerät (1) nach einem der Ansprüche 1 bis 13, **dadurch gekennzeichnet, dass** mindestens eine Detektionszelle einen Sensor umfasst, der aus der Liste {elektroakustischer Sensor, thermischer Sensor} ausgewählt ist.

15. Gerät (1) zum nicht-invasiven Analysieren eines Targets, das Folgendes umfasst:

- mindestens eine Intensitätsmodulationsvorrichtung, einen Lichtemitter, der ein intensitätsmoduliertes Licht mit einer regelbaren Modulationsfrequenz emittiert,
- mindestens eine Detektionszelle (12), die einen Sensor umfasst, der direkt oder indirekt eine Wärmewelle erfasst, die sich als Reaktion auf eine Bestrahlung des genannten Targets durch das emittierte Licht aus dem Target ausbreitet,
- mindestens ein Prozessormodul (13), das zum Empfangen und Verarbeiten von Sensordaten von mindestens einer Detektionszelle konfiguriert ist, wobei mindestens ein Lichtemitter konfiguriert ist zum:

a. Durchführen mindestens einer Bestrahlung des Targets mit dem Lichtemitter in einem ersten Modus, wobei die Modulationsfrequenz der Intensität des emittierten Lichts während jeder der mindestens einen Bestrahlung im ersten Modus über einen Frequenzdurchlaufbereich durchlaufen wird,
b. Durchführen mindestens einer zusätzlichen Bestrahlung des genannten Targets mit dem genannten Lichtemitter in dem genannten ersten Modus, wobei das mindestens eine Prozessormodul (13) konfiguriert ist zum:
c. Empfangen von Sensordaten von mindestens einer Detektionszelle für jede der mindestens einen Bestrahlung in einem ersten Modus,
d. Verarbeiten der Sensordaten, die für mindestens zwei Bestrahlungen in dem genannten ersten Modus empfangen wurden, und Bilden einer Zeitreihe, die Sensordaten für jede einer Vielzahl von diskreten Frequenzen in dem Frequenzdurchlaufbereich umfasst, und
e. Berechnen einer Diskretmodus-Modulationsfrequenz auf der Basis einer Analyse der Zeitreihe, die Daten für jede einer Vielzahl von diskreten Frequenzen in dem Frequenzdurchlaufbereich umfasst, wobei das genannte Analysieren das Bilden einer Zeitreihe für jede einer Vielzahl von diskreten Frequenzen und das Berechnen von mindestens einem Wert umfasst, der die Ähnlichkeit oder die Unähnlichkeit zwischen zwei Zeitreihen bei zwei der Vielzahl von diskreten Frequenzen anzeigt, und wobei:

- der mindestens eine Lichtemitter zum Durchführen mindestens einer Bestrahlung des Targets mit dem Lichtemitter in einem zweiten Modus konfiguriert ist, wobei das im zweiten Modus emittierte Licht mit der genannten von mindestens einem Prozessormodul berechneten Diskretmodus-Modulationsfrequenz intensitätsmoduliert wird, und
- das mindestens eine Prozessormodul zum Empfangen von Sensordaten von mindestens einer Detektionszelle für jede der mindestens einen Bestrahlung im zweiten Modus und zum Berechnen eines Analytwerts auf der Basis der für mindestens eine Bestrahlung im zweiten Modus empfangenen Sensordaten konfiguriert ist.

**16.** Gerät (1) zum nicht-invasiven Analysieren eines Targets nach Anspruch 15, **dadurch gekennzeichnet, dass** das Gerät tragbar ist.

**17.** System, das ein Gerät (1) zum nicht-invasiven Analysieren eines Targets nach einem der Ansprüche 15 bis 16 und eine Medikamentenabgabevorrichtung umfasst, wobei das Gerät zum nicht-invasiven Analysieren eines Targets ferner zum Senden von Daten zu der Medikamentenabgabevorrichtung konfiguriert ist, und das System ferner zum Bestimmen einer von der Medikamentenabgabevorrichtung zu gebenden Medikamententherapie auf der Basis von mindestens einem mit dem Prozessormodul des Geräts zum nicht-invasiven Analysieren eines Targets berechneten Analytwert konfiguriert ist.

## Revendications

**1.** Procédé d'analyse non invasive d'une cible avec un appareil d'analyse (1) comprenant :

- un dispositif de modulation d'intensité, un émetteur de lumière émettant une lumière modulée en intensité à une fréquence de modulation contrôlable,
- au moins une cellule de détection (12) comprenant un capteur détectant directement ou indirectement une onde thermique se propageant hors de la cible en réponse à une irradiation de ladite cible par la lumière émise,
- un module processeur (13) configuré pour recevoir et traiter des données de capteurs issues d'au moins une cellule de détection,

Dans lequel le procédé comprend :

a. effectuer au moins une irradiation de la cible avec l'émetteur de lumière dans un premier mode, la fréquence de modulation de l'intensité de la lumière émise étant balayée pendant chacune de la au moins une irradiation dans le premier mode sur une plage de balayage de fréquence, et le module processeur recevant des données de capteur provenant d'une cellule de détection pour chacune de la au moins une irradiation dans le premier mode,
b. effectuer au moins une irradiation supplémentaire de ladite cible avec ledit émetteur de lumière dans ledit premier mode, le module processeur recevant des données de capteur d'une cellule de détection pour chacune de la au moins une irradiation supplémentaire dans le premier mode,
c. traiter avec le module processeur (13) les données de capteur reçues pour au moins deux irradiations dans ledit premier mode et former avec le module processeur (13) une série temporelle comprenant des données de capteur pour chacune d'une pluralité de fréquences discrètes dans la plage de balayage de fréquence,
d. calculer, à l'aide du module processeur (13), une fréquence de modulation en mode discret sur la base d'une analyse de la série temporelle comprenant des données de capteur pour chacune d'une pluralité de fréquences discrètes dans la plage de balayage de fréquence, ladite analyse comprenant la formation d'une série temporelle pour chacune d'une pluralité de fréquences discrètes et le calcul d'au moins une valeur indicative de la similarité ou de la dissemblance entre deux séries temporelles à deux de la pluralité de fréquences discrètes,
e. réaliser au moins une irradiation de la cible avec un émetteur de lumière émettant une lumière modulée en intensité dans un deuxième mode, la fréquence de modulation de l'intensité de la lumière émise dans le deuxième mode étant ladite fréquence de modulation en mode discret calculée à l'étape d., le module processeur (13) recevant des données de capteur provenant d'une cellule de détection pour chacune de la ou des irradiations dans le deuxième mode,
f. calculer, à l'aide du module processeur (13), une valeur d'analyte sur la base des données de capteur reçues pour au moins l'une des irradiations dans le deuxième mode.

**2.** Procédé d'analyse non invasive d'une cible avec un appareil d'analyse (1) selon la revendication 1 **caractérisé en ce qu'**un premier capteur est prévu pour réaliser les étapes a et b et un deuxième capteur est prévu pour réaliser l'étape e.

3. Procédé d'analyse non invasive d'une cible avec un appareil d'analyse (1) selon la revendication 1 ou la revendication 2, **caractérisé en ce qu'**un deuxième émetteur de lumière émettant une lumière modulée en intensité à une fréquence de modulation contrôlable est prévu pour réaliser la au moins une irradiation à l'étape e.

4. Procédé d'analyse non invasive d'une cible avec un appareil d'analyse (1) selon l'une quelconque des revendications 1 à 3 **caractérisé en ce qu'**à l'étape e, au moins deux irradiations dans le deuxième mode sont effectuées, deux irradiations successives dans le deuxième mode étant séparées par une première période de temps TDM.

5. Procédé d'analyse non invasive d'une cible avec un appareil d'analyse (1) selon la revendication 4, **caractérisé en ce qu'**à l'étape f, la valeur de l'analyte est calculée sur la base des données de capteur reçues pour une pluralité des au moins deux irradiations dans le deuxième mode de l'étape e. précédente.

6. Procédé d'analyse non invasive d'une cible avec un appareil d'analyse (1) selon l'une quelconque des revendications 1 à 5 **caractérisé en ce que** les étapes b, c, d et e sont répétées périodiquement dans cet ordre, deux étapes b successives étant séparées par une seconde période de temps TSM.

7. Procédé d'analyse non invasive d'une cible avec un appareil d'analyse (1) selon l'une quelconque des revendications 4 à 5 et selon la revendication 6, **caractérisé en ce que** la première période de temps TDM est plus courte que ladite deuxième période de temps TSM.

8. Procédé d'analyse non invasive d'une cible avec un appareil d'analyse selon l'une quelconque des revendications 6 ou 7 **caractérisé en ce qu'**un premier nombre entier N est prédéterminé et qu'à l'étape c, la série temporelle est constituée avec les données acquises lors des N dernières irradiations en le premier mode.

9. Procédé d'analyse non invasive d'une cible avec un appareil d'analyse (1) selon la revendication 8 **caractérisé en ce qu'**à l'étape a, N-1 irradiations sont effectuées dans le premier mode, deux irradiations successives de l'étape a étant séparées par une troisième période de temps TDM.

10. Procédé d'analyse non invasive d'une cible avec un appareil d'analyse selon l'une quelconque des revendications 1 à 9, **caractérisé en ce qu'**à l'étape d, l'analyse de la série temporelle comprenant des données de capteur pour chacune d'une pluralité de fréquences discrètes dans la plage de balayage de fréquence comprend en outre l'exécution d'un algorithme de déformation temporelle dynamique et/ou une analyse de corrélation sur cette série temporelle.

11. Procédé d'analyse non invasive d'une cible avec un appareil d'analyse (1) selon l'une quelconque des revendications 1 à 10, **caractérisé en ce qu'**une lumière modulée en intensité comprend au moins une longueur d'onde qui est absorbée par un analyte à mesurer dans la cible.

12. Procédé d'analyse non invasive d'une cible avec un appareil d'analyse (1) selon la revendication 11, **caractérisé en ce que** la au moins une longueur d'onde se situe dans la plage infrarouge moyenne.

13. Procédé d'analyse non invasive d'une cible avec un appareil d'analyse (1) selon l'une quelconque des revendications 1 à 12, **caractérisé en ce que** l'appareil d'analyse comprend en outre une interface utilisateur ou est en outre configuré pour transmettre des données à un dispositif d'administration de médicament, et **caractérisé en ce qu'**à l'étape d, les résultats de l'analyse de la série temporelle comprenant des données de capteur pour chacune d'une pluralité de fréquences discrètes dans la plage de balayage de fréquence sont comparés à un seuil prédéterminé, et **en ce que**, sur la base du résultat de cette comparaison, un signal d'alarme est affiché sur l'interface utilisateur ou transmis au dispositif d'administration de médicament.

14. Procédé d'analyse non invasive d'une cible avec un appareil d'analyse (1) selon l'une quelconque des revendications 1 à 13 **caractérisé en ce qu'**au moins une cellule de détection comprend un capteur choisi dans la liste { capteur électroacoustique, capteur thermique}

15. Appareil (1) pour analyser de manière non invasive une cible comprenant :

- au moins un dispositif de modulation d'intensité, un émetteur de lumière émettant une lumière modulée en intensité à une fréquence de modulation contrôlable,
- au moins une cellule de détection (12) comprenant un capteur détectant directement ou indirectement une onde thermique se propageant hors de la cible en réponse à une irradiation de ladite cible par la lumière émise,
- au moins un module processeur (13) configuré pour recevoir et traiter des données de capteur provenant d'au moins une cellule de détection,

Dans lequel au moins un émetteur de lumière est configuré pour :

a. réaliser au moins une irradiation de la cible avec l'émetteur de lumière dans un premier mode, la fréquence de modulation de l'intensité de la lumière émise étant balayée lors de chacune de l'au moins une irradiation dans le premier mode sur une plage de balayage de fréquence,
b. effectuer au moins une irradiation supplémentaire de ladite cible avec ledit émetteur de lumière dans ledit premier mode,

dans lequel le au moins un module processeur (13) est configuré pour :

c. recevoir des données de capteur provenant d'au moins une cellule de détection pour chacune de l'au moins une irradiation dans un premier mode,
d. traiter les données de capteur reçues pour au moins deux irradiations dans ledit premier mode et former une série temporelle comprenant des données de capteur pour chacune d'une pluralité de fréquences discrètes dans la plage de balayage de fréquence, et
e. calculer une fréquence de modulation en mode discret sur la base d'une analyse de la série temporelle comprenant des données de capteur pour chacune d'une pluralité de fréquences discrètes dans la plage de balayage de fréquence, ladite analyse comprenant la formation d'une série temporelle pour chacune d'une pluralité de fréquences discrètes et le calcul d'au moins une valeur indicative de la similarité ou de la dissemblance entre deux séries temporelles à deux de la pluralité de fréquences discrètes,

et dans lequel

- le au moins un émetteur de lumière est configuré pour réaliser au moins une irradiation de la cible avec l'émetteur de lumière dans un deuxième mode, la lumière émise dans le deuxième mode étant modulée en intensité à ladite fréquence de modulation en mode discret calculée par au moins un module processeur, et
- le au moins un module processeur est configuré pour recevoir des données de capteur provenant d'au moins une cellule de détection pour chacune de la au moins une irradiation dans le deuxième mode et pour calculer une valeur d'analyte sur la base des données de capteur reçues pour au moins une irradiation dans le deuxième mode.

16. Appareil (1) pour analyser de manière non invasive une cible selon la revendication 15, **caractérisé en ce que** l'appareil est un wearable.

17. Système comprenant un appareil (1) pour analyser de manière non invasive une cible selon l'une quelconque des revendications 15 à 16 et un dispositif d'administration de médicament, l'appareil pour analyser de manière non invasive une cible étant configuré en outre pour envoyer des données au dispositif d'administration de médicament et le système étant de plus configuré pour déterminer une thérapie médicamenteuse à administrer par le dispositif d'administration de médicament sur la base d'une valeur d'analyte calculée par le module processeur de l'appareil pour analyser de manière non invasive une cible.

[Fig. 1]

[Fig. 2]

[Fig. 3]

[Fig. 4a]

Correlation at t1

Correlation at t2

Correlation at t3

Correlation OVER TIME

[Fig. 4b]

FIG. 4b1

FIG. 4b2

[Fig. 5]

[Fig. 6]

[Fig. 7a]

[Fig. 7b]

[Fig. 8a]

[Fig. 8b]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210052164 A
- US 20210109019 A
- US 10261011 B2
- WO 2020094265 A1

**Non-patent literature cited in the description**

- **ALLAN ROSENCWAIG ; ALLEN GERSHO.** Theory of the photoacoustic effect with solids. *Journal of Applied Physics,* 1976, vol. 47, 64
- **KOTTMANN J ; REY JM ; SIGRIST MW.** Mid-Infrared Photoacoustic Detection of Glucose in Human Skin : Towards Non-Invasive Diagnostics. *Sensors (Basel),* 10 October 2016, vol. 16 (10), 1663